(19)

Europäisches
Patentamt
European
Patent Office
Office européen
des brevets

(11) **EP 3 567 119 A1**

(12) **EUROPEAN PATENT APPLICATION**
published in accordance with Art. 153(4) EPC

(43) Date of publication:
**13.11.2019 Bulletin 2019/46**

(21) Application number: **18736382.5**

(22) Date of filing: **09.01.2018**

(51) Int Cl.:
*C12Q 1/68* (2018.01)     *G01N 33/574* (2006.01)
*G01N 33/68* (2006.01)     *A61K 31/11* (2006.01)
*A61K 31/167* (2006.01)     *A61K 48/00* (2006.01)

(86) International application number:
**PCT/KR2018/000395**

(87) International publication number:
**WO 2018/128516 (12.07.2018 Gazette 2018/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **09.01.2017 KR 20170002869**

(71) Applicant: **Met Life Sciences Co., Ltd.
Seoul 03722 (KR)**

(72) Inventors:
• **YOOK, Jong In
Seoul 06004 (KR)**
• **KIM, Hyun Sil
Seoul 03174 (KR)**
• **KIM, Nam Hee
Seoul 03174 (KR)**
• **CHOI, Ji Won
Seoul 01820 (KR)**
• **CHA, In Ho
Seoul 03174 (KR)**
• **ZHANG, Xianglan
Seoul 03981 (KR)**
• **KIM, Chang Sung
Seoul 06501 (KR)**

(74) Representative: **Mewburn Ellis LLP
City Tower
40 Basinghall Street
London EC2V 5DE (GB)**

(54) **PHARMACEUTICAL COMPOSITION FOR TREATING ORAL PRECANCER AND METHOD FOR PREDICTING OR DIAGNOSING ORAL PRECANCER OR ORAL CANCER**

(57) The present invention relates to: a method for providing information for the prediction or diagnosis of oral precancer or oral cancer characterized by confirming expression of the Axin2 gene or the Snail gene in an oral leukoplakia tissue sample or an oral submucous fibrosis tissue sample; and a use of a compound capable of delaying or inhibiting the progression of a precancerous lesion and recurrent oral cancer using the compound capable of effectively inhibiting the expression of the Axin2 gene or the Snail gene. According to the present invention, the possibility of oral leukoplakia or submucous fibrosis which causes white lesions to appear inside the oral cavity can be predicted and diagnosed, thereby being useful in eliminating the potential risks of oral cancer by early prediction of progression into oral precancer or oral cancer for which effective treatment does not currently exist.

FIG. 2

EP 3 567 119 A1

**Description**

[Technical Field]

The present invention relates to a method of predicting or diagnosing oral precancer or oral cancer, and more particularly, to a method of providing information for the prediction or diagnosis of oral precancer or oral cancer comprising Axin2 gene expression or snail (SNAI1) gene expression in a tissue sample of an oral leukoplakia or an oral submucous fibrosis, and a use of a compound for inhibiting Axin2 gene expression or Snail gene expression for delaying the progression of or inhibiting oral precancerous lesions and recurrent oral cancer.

[Background Art]

It is well known that oral leukoplakia with a white lesion in the oral cavity contains submucosal fibrosis and may develop malignancy (van der Waal I., Med Oral Patol Oral Cir Bucal.,19:e386, 2014; Holmstrup P et al., Oral Oncol, 42:461,2006; Holmstrup P., Oral Oncol 45:549, 2009). Oral leukoplakia occurs in about 1% of all age groups, and the malignancy rate of untreated lesions has been reported to be 2% to 3% per year (van der Waal I., Med Oral Patol Oral Cir Bucal.,19:e386, 2014). Meanwhile, the risk of recurrence after surgical treatment of non-homologous leukoplakia has been estimated up to 20%, and squamous cell carcinoma developed from leukoplakia has a low survival rate (Holmstrup P et al., Oral Oncol, 42:461, 2006). For the diagnosis of oral leukoplakia, many studies have been conducted to find predictive markers of malignancy including general pathologic examination, clinical parameters, and biomarkers, but the prediction rate is still limited.

Known risk factors for oral leukoplakia malignancy are age, female, long duration of leukoplakia, large size, medical history of head and neck cancer, non-homologous clinical appearance, the presence of histologic features of dysplasia, and leukoplakia lesions at the tongue edge or mouth bottom. In addition, predictive factors for oral leukoplakia malignancy are the presence of Candida albicans, the possibility of staining of leukoplakia lesions with toluidine blue, abnormal expression of numerous molecular markers including p16INK4a and Ki-67, chromosomal instability, and loss of heterozygosity in 9p and mutated TP53 (Abdulrahim MH et al., PLoS One, 8:e73738, 2013; Zhang L et al., Cancer Res, 65:8017, 2005; Nasser W et al., J Oral Pathol Med, 40:629, 2011; Siebers TJ et al., Oral Oncology 49:1121, 2013; Graveland AP et al., Oral Oncol, 49:1129, 2013). However, it is still difficult to accurately estimate potential risk factors for the development of oral squamous cell carcinoma (OSCC) with high reproducibility and certainty.

Epithelial-to-mesenchymal transition(EMT) silences epithelial cell characteristics and transforms the cell state into an invasive phenotype to exhibit mesenchymal characteristics (Dang TT et al., Cancer Res, 75:3925, 2015), and Snail1, which is a zinc-finger transcriptional inhibitor, plays an important role in EMT involving complementary morphogenesis in cancer. The snail1 protein is stabilized by the activation of the Wnt signal through the inhibition of serial phosphorylation of GSK-3. The Axin2 scaffolding protein, which is a typical transcription target for the TCF/LEF complex, regulates the nucleus cytoplasmic shuttle of GSK-3 to stabilize the nuclear Snail. Simultaneous expression of Axin2 and Snail1 in acute breast cancer and colorectal cancer is well known, but clinical significance and expression of the Wnt gene is not well known.

Although early EMT studies have focused primarily on metastatic processes, studies are now being conducted on the initial activation of EMT-related genes in preneoplastic lesions and non-invasive tumors. For example, a major pathogen of *Helicobacter pylori* in the human stomach strongly induces Snail-mediated EMT in gastric epithelial cells, and Snail1 is increased in non-invasive endometrial carcinoma. In addition, in EMT of oral leukoplakia, E-cadherin is decreased in moderate dysplasia during malignant processes, which may be determined as a high risk of malignancy (von Zeidler SV et al., BMC Cancer, 14:972, 2014). These results suggest that EMT gene expression in oral precancer has the potential to play a role in cancer progression.

In addition, unlike other cancers, in the case of oral cancer, there are many cases in which it is very difficult to perform surgical removal due to the presence of multiple precancerous lesions such as leukoplakia or erythroplakia by the field cancerization theory, and even after progressing to oral cancer, precancerous lesions and advanced cancer continue to recur (Slaughter DP et al, Cancer 1953, 6, 963-968; Mohan M & Jagannathan N, Oncology Rev 2014, 8:244, 13-19). In this case, proper therapeutic materials for precancerous lesions and advanced cancer have not yet been known, and most cases rely on surgical resection, but repeated surgical operations still have many limitations.

Therefore, as a result of having made intensive efforts to develop a method of diagnosing and treating the malignant development of oral leukoplakia, the inventors of the present invention confirmed that the progression and risk of oral epithelial cancer could be diagnosed, and when treated with a substance for inhibiting Axin2 and Snail, oral leukoplakia treatment was possible, thus completing the present invention.

[Disclosure]

[Technical Problem]

Therefore, the present invention has been made in view of the above problems, and it is an object of the present invention to provide a method of providing information for the prediction or diagnosis of oral precancer or oral cancer.

It is another object of the present invention to provide a kit for predicting or diagnosing oral precancer or oral cancer.

It is a further object of the present invention to provide a pharmaceutical composition for inhibiting the progression of oral precancer or oral cancer.

[Technical Solution]

In accordance with an aspect of the present invention, the above and other objects can be accomplished by the provision of a method of providing information for the prediction or diagnosis of oral precancer or oral cancer, the method comprising confirming an expression of the Axin2 gene or the Snail gene in a tissue sample of an oral leukoplakia or an oral submucous fibrosis.

In accordance with another aspect of the present invention, there is provided a composition for predicting or diagnosing oral precancer or oral cancer, the composition comprising an agent for confirming expression of the Axin2 gene or the Snail gene.

In accordance with another aspect of the present invention, there is provided a kit for predicting or diagnosing oral precancer or oral cancer, the kit comprising an agent for confirming expression of the Axin2 gene or the Snail gene.

In accordance with another aspect of the present invention, there is provided a pharmaceutical composition for delaying or inhibiting progression of an oral precancerous lesion and recurrent oral cancer, the pharmaceutical composition comprising, as an active ingredient, a compound for inhibiting expression of the Axin2 gene or the Snail gene.

In accordance with another aspect of the present invention, there is provided a method of delaying or inhibiting progression of an oral precancerous lesion and recurrent oral cancer, the method comprising administering a composition for inhibiting expression of the Axin2 gene or the Snail gene.

In accordance with another aspect of the present invention, there is provided a use of a compound for inhibiting expression of the Axin2 gene or the Snail gene for delaying or inhibiting progression of an oral precancerous lesion and recurrent oral cancer.

In accordance with another aspect of the present invention, there is provided a use of a compound for inhibiting expression of the Axin2 gene or the Snail gene for preparing a drug for delaying or inhibiting progression of an oral precancerous lesion and recurrent oral cancer.

[Description of Drawings]

FIG. 1 illustrates a method of characterizing the expression of Axin2 and Snail in tissues of patients with oral leukoplakia or oral submucous fibrosis.

FIG. 2 illustrates results of analyzing the expression of Axin2 and Snail in tissues of patients with oral leukoplakia or oral submucous fibrosis through immunohistochemical staining.

FIG. 3 illustrates the relationship between the expression of Axin2 and Snail and the basic information of clinical factors in tissues of patients with oral leukoplakia or oral submucous fibrosis.

FIG. 4 illustrates results of analyzing risk factors associated with cancer-free survivability among patients with Snail-positive oral leukoplakia.

FIG. 5 illustrates a nomogram for predicting 5-year, 10-year, and 15-year cancer-free survival rates using clinical parameters and the expression of Axin2 and Snail.

FIG. 6 illustrates nomograms predicting cancer-free survivability, plotted by analyzing clinicopathological parameters.

FIG. 7 illustrates a nomogram predicting cancer-free survivability, plotted by analyzing clinicopathological parameters.

FIG. 8 illustrates a decrease in the expression of Axin2 and Snail at an nM level by using niclosamide, which has almost no toxicity to patients and is a drug approved by the FDA.

FIG. 9 illustrates results of confirming whether the formation of an Axin-GSK3 complex was inhibited by niclosamide.

FIGS. 10A and 10B illustrate surface plasma resonance (SPR) analysis results to confirm whether niclosamide directly binds to GSK3, and FIG. 10C illustrates results of structural analysis of the Axin binding site of GSK3 and niclosamide.

FIG. 11 illustrates a decrease in Snail expression by 2'-Hydroxycinnamaldehyde (HCA), which is a major component of cinnamon, and a derivative thereof, i.e., 2'-benzoyloxycinnamaldehyde (BCA).

[Best Mode]

Unless defined otherwise, technical and scientific terms used herein have the same meaning as is commonly understood by one of ordinary skill in the art to which the present invention pertains. Generally, the nomenclature used herein is well known in the art and commonly used.

In the present invention, it was confirmed that Axin2 and Snail expression patterns were changed in oral leukoplakia malignancy, and it was confirmed whether Axin2 and Snail could be used as biomarkers for predicting oral leukoplakia malignancy. For immunohistochemical staining of Axin2 and Snail, oral mucosa samples of oral leukoplakia and oral submucous fibrosis, which were histopathologically confirmed, were used, and histological patterns of Axin2 and Snail were analyzed and classified. The correlation between clinicopathologic factors and malignancy of oral leukoplakia was confirmed by creating a nomogram graphically represented for 17 years and 10 months.

In one embodiment, the present invention relates to a method of providing information for the prediction or diagnosis of oral precancer or oral cancer, the method being characterized by confirming an expression level of the Axin2 gene or the Snail gene.

In the present invention, the confirmation of the expression level of the Axin2 gene or the Snail gene may be performed using an oral leukoplakia tissue sample or an oral submucous fibrosis tissue sample.

In the present invention, the confirmation of the expression level of the Axin2 gene or the Snail gene may be performed by comparing an expression level of the Axin2 gene or the Snail gene in lesion tissues derived from patients with oral leukoplakia or oral submucous fibrosis with that in tissues of healthy individuals.

In the present invention, the confirmation of the expression level of the gene may be performed using a method selected from the group consisting of RT-PCR, immunohistochemical staining, and enzyme-linked immunosorbent assay (ELISA).

In the present invention, the expression of Axin2 and Snail was evaluated in a long-term follow-up group of oral precancerous lesions.

The term "oral precancer" as used herein refers to a disease which can develop into oral cancer, and includes oral leukoplakia, oral erythroplakia, oral submucous fibrosis, and the like. Oral precancer patients include patients that have received surgical operations for oral cancer and persistent precancerous lesions and advanced cancer recurrence over several years according to the field cancerization theory.

In the present invention, the oral cancer may be squamous cell carcinoma, malignant lymphoma, sarcoma, malignant melanoma, or the like, preferably squamous cell carcinoma.

In the present invention, EMT genes were studied as a predictive role of malignant progression of oral precancer. Although many genes contain Canonical Wnt signals, we have studied the GSK-3 scaffolding protein Axin2 and the E-cadherin transcription inhibitor Snail to assess the potential of this biomarker.

In the absence of the Wnt signal, GSK3 degrades $\beta$-catenin and plays an inhibiting role in maintaining an epithelial phenotype. Snail inhibits the transcription of the E-cadherin gene to express a mesenchymal phenotype. In addition, Snail activity is inhibited by selective phosphorylation of GSK3 (Carver EA et al., Mol Cell Biol, 21:8184, 2001; Zhou BP et al., Nat Cell Biol, 6:931-40, 2004; 21. Bachelder RE et al., J Cell Biol, 168:29, 2005; Yook JI et al., J Biol Chem, 280:11740, 2005).

In the present invention, the presence of Axin2 is closely associated with the malignant progression of oral precancerous lesions. Considering that Axin2 is a typical downstream factor in the canonical Wnt pathway, results indicate that increased Wnt activity may play an important role in the malignancy of oral leukoplakia.

In one embodiment of the present invention, it was confirmed that, while the normal mucosal epithelium exhibits the cytoplasmic expression of Axin2 and Snail, Axin2 and Snail were not expressed in the nucleus. It was also confirmed that, compared to a normal control, Snail of the epithelium was significantly expressed in both the nucleus and cytoplasm in oral leukoplakia lesions.

In another embodiment of the present invention, a biopsy sample of oral submucous fibrosis was further examined. Oral submucous fibrosis is a chronic progressive fibrous mucosal disease of the oral cavity and has a potential risk of malignancy. Immunohistochemical staining studies revealed similar expression patterns of Axin2 and Snail in epithelial lesions of oral submucous fibrosis.

However, Axin2 has been found to act as a tumor gene by promoting Snail-mediated EMT and metastatic activity during cancer progression (Klar M et al., Breast Cancer Res Treat, 112:523, 2008). In addition, reducing Axin2 expression decreases Snail activity and returns EMT, thereby inhibiting invasive and metastatic tumor activity (Klar M et al., Breast Cancer Res Treat, 112:523, 2008).

In the present invention, it was confirmed from clinicopathological data that there was no significant association between tissue Axin2 expression of oral leukoplakia and clinical variables, and when a Snail-positive oral leukoplakia patient group was reevaluated, excellent Axin2 expression was considerably associated with the risk of malignancy. Interestingly, patients with oral submucous fibrosis showed patterns similar to clinicopathological association with regard to the risk of malignancy. Furthermore, in our studies, univariate and multivariate analyses have shown that gender, age, Snail expression, and Axin2 expression are significantly associated with the risk of malignancy of oral leukoplakia patients. In particular, Axin2 overexpression was found to be the most important factor for predicting the risk of malignancy in Snail-positive oral leukoplakia patients. Therefore, it is concluded that Snail activity is caused by the loss of function of the scaffolding protein Axin2 and the inactivation of destructive complexes during malignant processes in patients with oral leukoplakia.

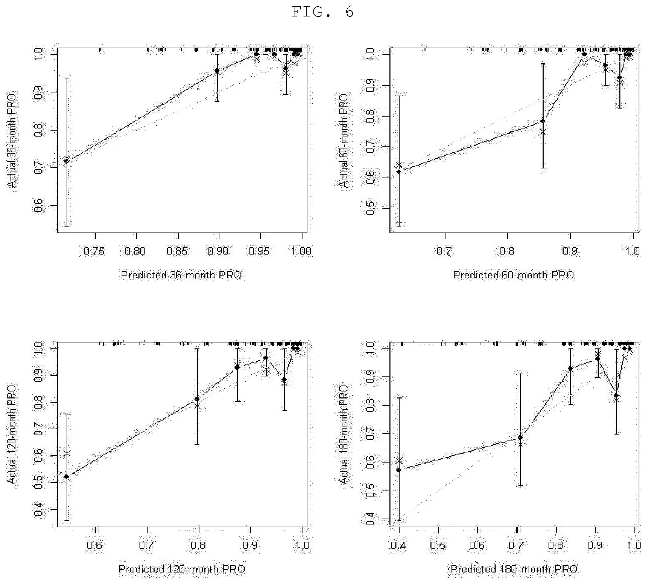

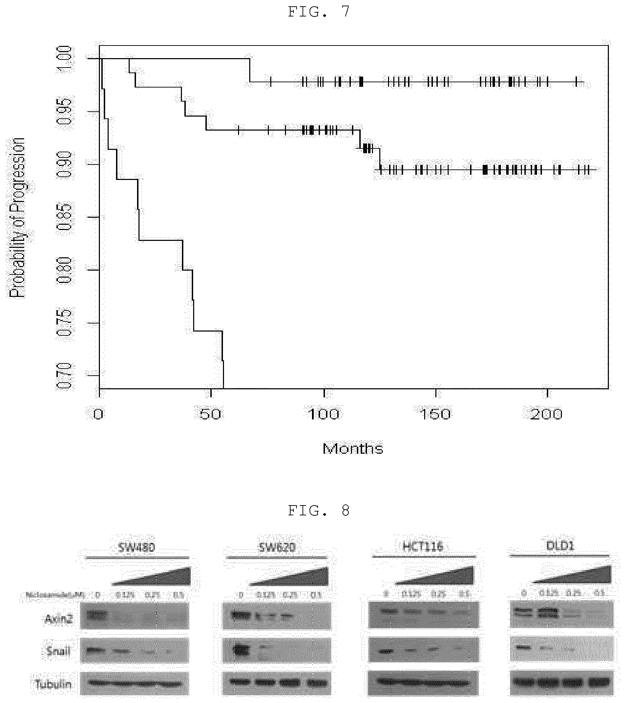

In the present invention, based on cross-sectional and retrospective cohort study results, a nomogram was created to evaluate the risk factors of malignancy of oral leukoplakia. Nomograms of FIGS. 6 and 7 were created by analyzing clinicopathological variables through follow-up for 17 years and 10 months. The nomograms predicting cancer-free survivability for 5 years, 10 years, and 15 years in patients with pathologically confirmed oral leukoplakia were created, and when containing Axin2 expression and Snail1 expression, c-index was shown to be 0.790. These results support that the expression of Axin2 and Snail may be used as important biomarkers capable of predicting malignant progression in oral leukoplakia.

The present invention provides predictive values of Axin2 and Snail expression which are predictive of oral leukoplakia developing into oral squamous epithelial cell carcinoma. Since there is no effective treatment for malignancy, biomarker investigation for risk prediction is a method to improve cancer-free survival time in patients with oral leukoplakia. In addition, to predict malignancy in other oral mucosal lesions having a potential risk of malignancy such as oral submucous fibrosis, it is useful to evaluate expression patterns of Axin2 and Snail in tissues.

In another embodiment, the present invention relates to a composition or kit for predicting or diagnosing oral precancer or oral cancer, which includes an agent for confirming the expression of the Axin2 gene or the Snail gene.

In the present invention, the agent for confirming the expression of the gene may be selected from the group consisting of an antibody or aptamer that specifically binds to the Axin2 protein or the Snail protein, a primer that specifically amplifies the Axin2 gene or the Snail gene, and a probe specifically binding to the Axin2 gene or the Snail gene.

In the method of the present invention, to provide information that is useful for the prediction or diagnosis of oral precancer or oral cancer, a method of measuring an expression level of the gene may be performed by, preferably, genetic analysis.

When the present invention is practiced based on genetic analysis, primers or probes that specifically bind to the listed target gene sequences are used. In the present invention, when primers are used, gene amplification is performed to examine an expression level of the target gene. Since the present invention analyzes the expression level of a gene, the expression level of the gene is determined by examining an mRNA expression level of a target gene in a sample (e.g., a cell sample or tissue sample) to be analyzed. Thus, in the present invention, the gene amplification reaction may be, in principle, performed using mRNA in a sample as a template and a primer that binds to mRNA or cDNA. To obtain mRNA, total RNA is first separated from a sample. The isolation of total RNA may be carried out according to a general method known in the art (see Sambrook, J. et al., Molecular Cloning. A Laboratory Manual, 3rd ed. Cold Spring Harbor Press(2001); Tesniere, C. et al., Plant Mol. Biol. Rep., 9:242(1991); Ausubel, F.M. et al., Current Protocols in Molecular Biology, John Willey & Sons(1987); and Chomczynski, P. et al., Anal. Biochem. 162:156(1987)). For example, TRIzol may be used to readily isolate total RNA in a cell. Next, cDNA is synthesized from the separated mRNA, and this cDNA is amplified. Since the total RNA of the present invention is isolated from a human sample, it has a poly-A tail at the end of the mRNA, and cDNA may be easily synthesized using oligo dT primer and reverse transcriptase by using such a sequence characteristic (see PNAS USA, 85:8998(1988); Libert F, et al., Science, 244:569(1989); and Sambrook, J. et al., Molecular Cloning. A Laboratory Manual, 3rd ed. Cold Spring Harbor Press (2001)). Subsequently, the synthesized cDNA is amplified through gene amplification reaction.

The primer used in the present invention is hybridized or annealed at one site of the template to form a double-stranded structure. Conditions for nucleic acid hybridization suitable for forming such a double-stranded structure are described in Joseph Sambrook et al., Molecular Cloning, A Laboratory Manual, Cold Spring Harbor Laboratory Press, Cold Spring Harbor, NY.(2001) and Haymes, B.D., et al., Nucleic Acid Hybridization, A Practical Approach, IRL Press, Washington, D.C. (1985).

A variety of DNA polymerases may be used in the amplification of the present invention, and examples thereof include a Klenow fragment of *E. coli* DNA polymerase I, a thermostable DNA polymerase, and bacteriophage T7 DNA polymerase. Preferably, the polymerase is a thermostable DNA polymerase obtainable from a variety of bacterial species, and examples thereof include Thermus aquaticus(Taq), Thermus thermophilus(Tth), Thermus filiformis, Thermis flavus, Thermococcus literalis, and Pyrococcus furiosus(Pfu). When a polymerization reaction is performed, excess amounts of components needed for the reaction may be supplied to a reaction vessel. Excess amounts of components needed for amplification reaction refer to amounts to which the amplification reaction is not substantially restricted to concentrations of the components.

It is required to add a cofactor such as Mg2+, dATP, dCTP, dGTP, and dTTP to a reaction mixture to an extent to which a desired amplification degree can be achieved. All enzymes used in the amplification reaction may be active under the same reaction conditions. In fact, buffer enables all enzymes to have optimal reaction conditions. Thus, the amplification process of the present invention may be performed on a single reactant without changing conditions such as the addition of reactants.

In the present invention, annealing or hybridization is carried out under stringent conditions that allow specific binding between a target nucleotide sequence and a primer. The stringent conditions for annealing are sequence-dependent and vary according to environmental variables.

The term "amplification reaction" as used herein refers to a reaction for amplifying a nucleic acid molecule. Various amplification reactions have been reported in the art, and include polymerase chain reaction (hereinafter referred to as PCR) (US Patent Nos. 4,683,195, 4,683,202, and 4,800,159), reverse transcription-PCR (hereinafter referred to as RT-PCR) (Sambrook et al., Molecular Cloning. A Laboratory Manual, 3rd ed. Cold Spring Harbor Press (2001)), Miller, H. I.(WO 89/06700) and Davey, C. et al. (EP 329,822), ligase chain reaction (LCR) (17, 18), Gap-LCR (WO 90/01069), repair chain reaction (EP 439,182), transcription-mediated amplification (TMA) (19) (WO 88/10315), self-sustained sequence replication(20) (WO 90/06995), selective amplification of target polynucleotide sequences (US Patent No. 6,410,276), consensus sequence primed polymerase chain reaction (CP-PCR)(US Patent No. 4,437,975), arbitrarily primed polymerase chain reaction (AP-PCR) (US Patent Nos. 5,413,909 and 5,861,245), nucleic acid sequence based amplification (NASBA) (US Patent Nos. 5,130,238, 5,409,818, 5,554,517, and 6,063,603), strand displacement amplification, and loop-mediated isothermal amplification (LAMP), but the present invention is not limited thereto.

Other amplification methods that may be used are described in U.S. Patent Nos. 5,242,794, 5,494,810, 4,988,617 and 09/854,317. In the most preferred embodiment of the present invention, the amplification process is performed according to polymerase chain reaction (PCR) disclosed in US Patent Nos. 4,683,195, 4,683,202, and 4,800,159.

PCR is the most well-known nucleic acid amplification method, and many variations and applications thereof have been developed. For example, touchdown PCR, hot start PCR, nested PCR, and booster PCR have been developed by modifying traditional PCR procedures to enhance the specificity or sensitivity of PCR. In addition, real-time PCR, differential display PCR (DD-PCR), rapid amplification of cDNA ends (RACE), multiplex PCR, inverse polymerase chain reaction (IPCR), vectorette PCR, thermal asymmetric interlaced PCR (TAIL-PCR), and multiplex PCR have been developed for specific applications. For more information on PCR, see McPherson, M.J., and Moller, S.G. PCR. BIOS Scientific Publishers, Springer-Verlag New York Berlin Heidelberg, N.Y. (2000), the teachings of which are incorporated herein by reference. The term "primer" as used herein refers to a single-stranded oligonucleotide capable of acting as a starting point of template-directed DNA synthesis under suitable conditions (i.e., four other nucleoside triphosphates and polymerase) in a suitable buffer at an appropriate temperature. The suitable length of the primer is typically 15-30 nucleotides, although it varies depending on various factors such as temperature and use of the primer. Short primer molecules generally require lower temperatures to form sufficiently stable hybrid complexes with the template.

The sequence of the primer does not need to have a sequence completely complementary to a partial sequence of the template, and it is sufficient when the sequence of the primer has sufficient complementary within a range that enables the primer to be hybridized with the template and implement its intrinsic function. Thus, a primer pair of the present invention does not need to have a sequence completely complementary to the sequence of the target gene as a template, and it is sufficient as long as it has sufficient complementary within a range that enables the primer pair to be hybridized with the sequence of the target gene to act as a primer. The primer of the present invention may be prepared with a sequence complementary to the mRNA (i.e., cDNA) sequence of the target gene. Design of such a primer may be easily carried out by those of ordinary skill in the art with reference to the cDNA sequence of the target gene.

cDNA of the amplified target gene is analyzed using a suitable method to examine the expression level of the gene. For example, the above-described amplification reaction product may be subjected to gel electrophoresis, and the resulting band may be observed and analyzed to examine the expression level of each gene. Through these amplification reactions, when the expression of each gene is higher than that of normal individuals, the possibility of developing into oral cancer or oral cancer may be predicted.

In addition, the possibility of developing into oral cancer or oral precancer may also be predicted by hybridization-based analysis using a probe hybridized to the target gene or cDNA thereof. The term "probe" as used herein refers to a single-stranded nucleic acid molecule and includes a sequence complementary to a target nucleic acid sequence. According to one embodiment of the present invention, the probe of the present invention may be modified within a range that does not impair an advantage of the probe, i.e., the improvement of hybridization specificity. Such a modification, i.e., labeling may provide a signal for detecting hybridization, which may be linked to an oligonucleotide. Suitable labels include, but are not limited to, fluorescent moieties (e.g., fluorescein), phycoerythrin, rhodamine, lissamine, Cy3 and Cy5 (Pharmacia), chromophores, chemiluminescent moieties, magnetic particles, radioactive isotopes (P32 and S35), mass labels, electron dense particles, enzymes (alkaline phosphatase or horseradish peroxidase), cofactors, substrates for enzymes, heavy metals (e.g., gold), antibodies, streptavidin, biotin, and hapten having a specific binding partner, such as digoxigenin and a chelating group. Labeling may be performed using various methods commonly used in the art, e.g., a nick translation method, a random priming method (Multiprime DNA labelling systems booklet, "Amersham"(1989)), and a carnation method (Maxam & Gilbert, Methods in Enzymology, 65:499(1986)). The label provides a signal that can be detected using fluorescence, radiation, coloring measurement, weight measurement, X-ray diffraction or absorption, magnetism, enzymatic activity, mass analysis, binding affinity, high frequency for hybridization, or nanocrystals.

According to one embodiment of the present invention, the probe of the present invention that is hybridized to target gene cDNA may be immobilized onto an insoluble carrier (e.g., a nitrocellulose or nylon filter, a glass plate, a silicon support, and a fluorocarbon support) to be prepared as a microarray. In the microarray, the probe of the present invention is used as a hybridizable array element. The immobilization onto the insoluble carrier is performed using a chemical bonding method or a covalent bonding method such as UV. For example, the hybridizable array element may bind to a glass surface modified to include an epoxy compound or an aldehyde group, and may also bind to a polylysine coating surface by UV. In addition, the hybridizable array element may be bound to a carrier via a linker (e.g., an ethylene glycol oligomer and a diamine). cDNA of each target gene may be obtained by the above-described process. The hybridization-based analysis may also be performed by labeling cDNA of the gene instead of using the probe. When the probe is used, the probe is hybridized with a cDNA molecule. In the present invention, suitable hybridization conditions may be determined through a series of procedures by an optimization procedure. For example, conditions such as temperature, the concentration of a component, hybridization and washing time, buffer components, and pH and ionic intensity thereof, and the like depend on various factors such as the length of a probe, GC content, a target nucleotide sequence, and the like. The detailed conditions for hybridization may be confirmed in Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999). After the hybridization reaction, a hybridization signal generated through the hybridization reaction is detected. Hybridization signaling may be performed using various methods depending on the type of label bound to the probe. For example, when the probe is labeled with an enzyme, a substrate of the enzyme may be reacted with a hybridization reaction product to confirm the presence or absence of hybridization. Suitable combinations of enzyme/substrate include a peroxidase (e.g., horseradish peroxidase) and chloronaphthol, aminoethylcarbazole, diaminobenzidine, D-luciferin, bis-N-methylacridinium nitrate (lucigenin), resorufin benzyl ether, luminol, an Amplex red reagent (10-acetyl-3,7-dihydroxyphenoxazine), p-phenylenediamine-HCl and pyrocatechol (HYR), tetramethylbenzidine (TMB), 2,2'-Azine-di[3-ethylbenzthiazoline sulfonate] (ABTS), o-phenylene diamine (OPD), and naphthol/pyronine; alkaline phosphatase and bromochloroindolyl phosphate (BCIP), nitroblue tetrazolium (NBT), naphthol-AS-B1-phosphate and ECF substrate; glucose oxidase and nitroblue tetrazolium (t-NBT); and the like. When the probe is labeled with gold particles, this may be detected by a silver staining method using silver nitrate. By analyzing the intensity of the hybridizable signal by the above-described hybridization process, the possibility of developing into oral precancer or oral cancer may be predicted. That is, when a signal for cDNA of each target gene in a sample is stronger than an expression level thereof in normal individuals, the possibility of developing into oral precancer or oral cancer may be predicted.

The kit provided in the present invention may optionally include a reagent needed for performing target amplification PCR (e.g., PCR), such as buffer, a DNA polymerase cofactor, and deoxyribonucleotide-5-triphosphate. The kit of the present invention may also optionally include various polynucleotide molecules, reverse transcriptase, various buffers and reagents, and antibodies that inhibit DNA polymerase activity, which are needed for performing asymmetric PCR, and for melt curve analysis using a liquid-type array, the kit may also include lambda exonuclease, various buffers and reagents, and microwell plates.

In addition, in the kit, an optimal amount of a reagent used in a specific reaction may be easily determined by one of ordinary skill in the art that has acquired the teachings disclosed herein, and the kit may be designed as a separate package or compartment including the aforementioned components.

The present invention also provides a low molecular weight compound for inhibiting the expression of Axin2 and Snail that promotes cancer progression of precancerous lesions, thus effectively treating and preventing oral precancerous lesions and recurrent oral cancer that are characterized by repeated recurrence and field cancerization.

Therefore, in another embodiment, the present invention relates to a pharmaceutical composition for delaying or inhibiting the progression of oral precancerous lesions and recurrent oral cancer, which includes a compound for inhibiting the expression of the Axin2 gene or the Snail gene, as an active ingredient.

In another embodiment, the present invention relates to a method of delaying or inhibiting the progression of oral precancerous lesions and recurrent oral cancer, including administering a compound for inhibiting the expression of the Axin2 gene or the Snail gene.

In another embodiment, the present invention relates to a use of a compound for inhibiting the expression of the Axin2 gene or the Snail gene for delaying or inhibiting the progression of oral precancerous lesions and recurrent oral cancer.

In another embodiment, the present invention relates to a use of a compound for inhibiting the expression of the Axin2 gene or the Snail gene in preparing a drug for delaying or inhibiting the progression of oral precancerous lesions and recurrent oral cancer.

In the present invention, the compound for inhibiting the expression of the Axin2 gene or the Snail gene may be selected from the group consisting of niclosamide, 2'-hydroxycinnamaldehyde (HCA), 2'-benzoyloxycinnamaldehyde (BCA) as a derivative of HCA, and a pharmaceutically acceptable salt thereof.

The composition of the present invention is prepared in the form of a solution, a powder, a gel, or a patch. When administered to a patient, precancerous lesions and recurrent oral cancer may be effectively controlled without side effects.

A carrier used in the pharmaceutical composition of the present invention includes a pharmaceutically acceptable carrier, an adjuvant, and a vehicle, and thus is collectively referred to as a pharmaceutically acceptable carrier. Examples of pharmaceutically acceptable carriers that may be used in the pharmaceutical composition of the present invention include, but are not limited to, ion exchange resins, alumina, aluminum stearate, lecithin, serum proteins (e.g., human serum albumin), buffer substances (e.g., various phosphates, glycine, sorbic acid, potassium sorbate, and partial glyceride mixtures of saturated vegetable fatty acids), water, salts or electrolytes (e.g., protamine sulfate, disodium hydrogen phosphate, potassium hydrogen phosphate, sodium chloride, and zinc salts), colloidal silica, magnesium trisilicate, polyvinyl pyrrolidone, cellulose-based substrates, polyethylene glycol, sodium carboxymethylcellulose, polyarylates, waxes, polyethylene-polyoxypropylene-blocking polymers, polyethylene glycol, and lanolin.

Administration routes of the pharmaceutical composition according to the present invention include, but are not limited to, oral administration, intravenous administration, intramuscular administration, intraarterial administration, intramedullary administration, intrathecal administration, intracardiac administration, transdermal administration, subcutaneous administration, intraperitoneal administration, intranasal administration, intestinal administration, topical administration, sublingual administration, and intrarectal administration.

The administration routes may be oral administration and parenteral administration. The term "parenteral" as used herein includes subcutaneous, intradermal, intravenous, intramuscular, intraarticular, intrasynovial, intrasternal, intrathecal, intralesional and intracranial injection or infusion techniques.

The pharmaceutical composition may be in the form of a sterile injectable preparation, either as a sterile injectable aqueous or oil-based suspension. The suspension may be formulated according to techniques known in the art using a suitable dispersing or wetting agent (e.g., Tween 80) and a suspending agent. The sterile injectable preparation may also be a sterile injectable solution or suspension in a non-toxic parenterally acceptable diluent or solvent (e.g., a solution in 1,3-butanediol). Pharmaceutically acceptable vehicles and solutions may include mannitol, water, a Ringer's solution, and an isotonic sodium chloride solution. In addition, sterile nonvolatile oil is generally used as a solvent or a suspending medium. For this purpose, any non-volatile oil with low irritation, including synthetic mono- or diglycerides may also be used. Fatty acids such as oleic acid and glyceride derivatives thereof are useful in injectable preparations as well as pharmaceutically acceptable natural oils (e.g., olive oil or castor oil), especially polyoxyethylated ones thereof.

The pharmaceutical composition of the present invention may be orally administered in any orally acceptable dosage form, by including, but being not limited to, capsules, tablets, and aqueous suspensions, and solutions. In the case of oral tablets, commonly used carriers include lactose and corn starch. Lubricants such as magnesium stearate are also typically added. For oral administration in a capsule form, useful diluents include lactose and dried corn starch. When the aqueous suspension is orally administered, the active ingredient is combined with an emulsifying agent and a suspending agent. If needed, a sweetening agent and/or a flavoring agent and/or a colorant may be added.

The pharmaceutical composition of the present invention may also be administered in the form of suppositories for rectal administration. These compositions may be prepared by mixing the compounds of the invention with suitable non-polar excipients which are solid at room temperature but liquid at rectal temperature. Such materials include, but are not limited to, cocoa butter, beeswax, and polyethylene glycols.

Oral administration of the pharmaceutical composition according to the present invention is particularly useful when desired treatment is associated with a site or organ that is accessible by topical application. When topically applied to the skin, the pharmaceutical composition should be formulated as a suitable ointment including the active ingredient suspended or dissolved in a carrier. Carriers for topical administration of the compound of the present invention include, but are not limited to, mineral oil, liquid paraffin, white Vaseline, propylene glycol, polyoxyethylene, polyoxypropylene compounds, emulsifying wax, and water. In other embodiments, the pharmaceutical composition may be formulated into suitable lotions or creams including an active compound suspended or dissolved in a carrier. Examples of suitable carriers include, but are not limited to, mineral oil, sorbitan monostearate, polysorbate 60, cetyl ester wax, stearyl alcohol, 2-octyldodecanol, benzyl alcohol, and water. The pharmaceutical composition of the present invention may also be topically applied in the form of rectal suppositories as a suitable enema to the lower intestinal tract. Topically applied transdermal patches are also included in the present invention.

The pharmaceutical composition of the present invention may be administered intranasally in the form of aerosols or inhaled. Such compositions may be prepared according to techniques well known in the art of pharmaceuticals and may be prepared as a solution in salt water using benzyl alcohol or other suitable preservatives, an absorption enhancer to enhance bioavailability, fluorocarbons, and/or other solubilizing or dispersing agents known in the art.

The compound of the present invention may be used in combination with a general anti-inflammatory agent or in combination with a matrix metalloproteinase inhibitor, a lipoxygenase inhibitor, and inhibitors of cytokines other than IL-1β. The compound of the present invention may also be used in combination with immunomodulators (e.g., bropirimines, anti-human alpha interferon antibodies, IL-2, GM-CSF, methionine enkephalin, interferon alpha, diethyldithiocarbamate, tumor necrosis factor, naltrexone, and rEPO) or prostaglandins, to prevent or eradicate IL-1-mediated symptoms such as inflammation. When the compound of the present invention is administered in combination with other therapeutic agents, these may be sequentially or simultaneously administered to a patient.

The term "therapeutically effective amount" as used herein refers to a dosage level of about 1 mg/kg/day to about 100 mg/kg/day (typically, about 60 mg/patient/day to about 6 g/patient/day) to be used in treatment of the symptoms in humans.

The term "prophylactically effective amount" as used herein refers to a dosage level of about 0.1 mg/kg/day to about 100 mg/kg/day (typically, about 6 mg/patient/day to about 6 g/patient/day) to be used in prevention of the symptoms in humans.

However, it will be understood that a specific effective amount for a particular patient may vary depending on various factors, including the activity of particular compound used, age, body weight, general health conditions, gender, diet, administration time, administration routes, excretion rate, drug combination, and the severity of a particular disease to be prevented or treated. The pharmaceutical composition according to the present invention may be formulated into pills, sugar-coated tablets, capsules, liquids, gels, syrups, slurries, and suspensions.

In an exemplary embodiment, the pharmaceutical composition for oral administration may be prepared by mixing the active ingredient with solid excipients and may be prepared in the form of granules for preparation in the form of tablets or sugar-coated tablets. Suitable excipients include sugars such as lactose, sucrose, mannitol, and sorbitol or starches from corn, wheat flour, rice, potatoes, or other plants, cellulose such as methylcellulose, hydroxypropyl methyl cellulose, or sodium carboxymethylcellulose, carbohydrates or gelatin such as gums including gum arabic and gum tragacanth, or protein fillers such as collagen. If needed, disintegrating or solubilizing agents in the form of respective salts such as cross-linked polyvinylpyrrolidone, agar and alginic acid or sodium alginic acid may be added.

In an exemplary embodiment, for parenteral administration, the pharmaceutical composition of the present invention may be prepared as a water-soluble solution. Preferably, physically suitable buffer solutions such as Hank's solution, Ringer's solution, or physically buffered saline may be used. Water-soluble injection suspensions may include a substrate capable of increasing the viscosity of a suspension, such as sodium carboxymethylcellulose, sorbitol, or dextran. In addition, suspensions of the active ingredient may be prepared into suitable oily injection suspensions. Suitable lipophilic solvents or carriers include fatty acids such as sesame oil or synthetic fatty acid esters such as ethyl oleate, triglycerides, or liposomes. Polycationic non-lipid amino polymers may also be used as carriers. Optionally, the suspension may use a suitable stabilizing agent or drug to increase the solubility of the compound and to prepare a highly concentrated solution.

Examples

Hereinafter, the present invention will be described in further detail with reference to the following examples. It will be obvious to those of ordinary skill in the art that these examples are provided only to more particularly describe the present invention and are not intended to limit the scope of the present invention in accordance with the essence of the present invention.

Example 1: Characterization of Expression of Axin2 and Snail in Oral Leukoplakia

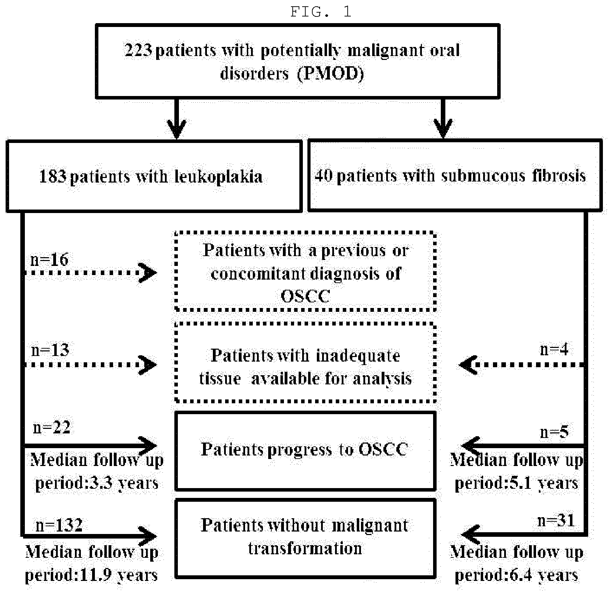

Oral leukoplakia tissue samples used in the examples of the present invention were supplied from the pathology department of the dental hospital of Yonsei Medical Center, and oral submucous fibrosis tissue samples were supplied from the department of oral pathology of Peradeniya Dental University, Sri Lanka. Between 1995 and 2010, biopsy specimens were obtained from 223 patients with potentially malignant oral disorders (PMOD). Patients who were previously or concomitantly diagnosed with OSCC (n=16) or with tissues inappropriate for analysis (n=17) were excluded from the analysis. The analysis was performed on a total of 154 patients (96 males and 59 females; mean age of 55; age range between 13 years old and 89 years old) and 36 patients with oral submucous fibrosis (mean follow-up period: 6.4 years) (24 males and 12 females; mean age of 45; age range between 21 years old and 75 years old) (see FIG. 1).

Characteristics of the tissue sample donors are shown in Table 1.

[Table 1]

| Clinical variables | NOM (%) | OL (%) | SMF (%) |
|---|---|---|---|
| Total sample | 18 | 154 | 36 |

(continued)

| Clinical variables | NOM (%) | OL (%) | SMF (%) |
|---|---|---|---|
| Mean age(years old) | 55 | 45 | |
| Gender | | | |
| Male | 6 (33.3) | 96(62.3) | 24(66.7) |
| Female | 12 (66.7) | 58(37.7) | 12(33.3) |
| Site | | | |
| Oral mucosa | 0(0) | 44(28.6) | 32(88.9) |
| Tongue | 0(0) | 42(27.3) | 1(2.8) |
| Gum | 18(100) | 68(44.2) | 0(0) |
| Lips | 0(0) | 0(0) | 2(5.6) |
| Unidentified | 0(0) | 0(0) | 1(2.8) |

Oral leukoplakia and oral submucous fibrosis of sample slides were reviewed and diagnosed by pathologists. Normal oral mucosal tissues to be used as a normal control were supplied from 18 individuals with 3rd molar extraction (6 males and 12 females; mean age of 45; age range between 11 years old and 53 years old) and used. This analysis was carried out under the approval of the Research Advisory Committee (IRB 2-2013-0045) of the Dental College of Yonsei University.

To confirm the expression of Axin2 and Snail, immunohistochemical staining was performed as follows.

The tissue samples were deparaffinized with xylene and the alcohols were rehydrated, and then the tissue sections were allowed to react in a mixture of $H_2O_2$ and methanol diluted 1:40 at room temperature for 10 minutes to block endogenous peroxidase activity. Antigen retrieval was performed using a pressure-cooking (continuous cooking) method in antigen retrieval buffer (Dako, Glostrup, Denmark). After blocking with 5% bovine serum albumin at room temperature for 5 minutes, the sections were allowed to react at room temperature for 2 hours with a monoclonal rabbit anti-human Axin2 IgG (Abcam, Cambridge, UK) diluted at a ratio of 1:200 and monoclonal mouse anti-human Snail1 IgG (Abcam) diluted at a ratio of 1:500. A real envision HRP rabbit/mouse detection system (Dako) was used as a secondary antibody. The resulting sections were treated with 3,3'-diaminobenzidine, and then stained with hematoxylin. A negative control was treated with a rabbit anti-human IgG antibody (R&D Systems, Minneapolis, MN, USA) or a mouse anti-human IgG (Dako) antibody as a primary antibody, and sections without positive cells were considered negative. SW480 cell blots were used as positive controls for both Axin2 and Snail1. In particular, the scaffolding protein Axin2 and the transcription factor Snail1 were found in the cytoplasm and nucleus, respectively. Axin2 immunoreactivity and Snail immunoreactivity were respectively divided into low and high Axin2 expression groups and low and high Snail expression groups due to the absence of the upper two thirds of the epidermis in the oral mucosa. Low expression indicates specimens expressing limited positively-stained cells in the lower one third of the epidermis or no positive cells, and high expression indicates specimens expressing benign cells of the upper two thirds of the epidermis.

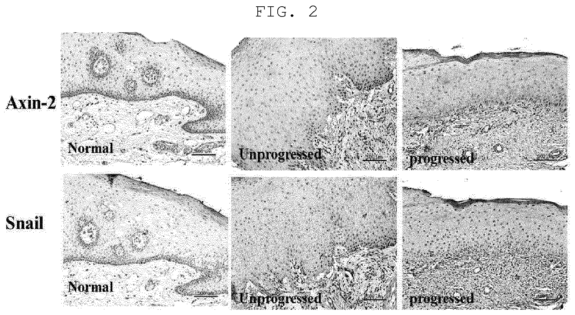

The results thereof are illustrated in FIG. 2. In normal oral mucosa, cytoplasmic Axin2 expression was found in mucosal epithelial cells in 3 of 18 healthy specimens as a control (16.7%), and cytoplasmic Snail1 expression was found in 5 specimens (27.8%) (see FIGS. 2A and 2D). In contrast, compared to the healthy control, significantly increased cytoplasmic Axin2 expression was confirmed in epithelial cells of 107 of 154 oral leukoplakia patients (69.5%; P < 0.001) and 25 of 36 oral submucous fibrosis patients (69.4%; P < 0.001) (see FIGS. 2B and 2E). Overall tissue immunoreactivity for Axin2 was high in 19 oral leukoplakia patients (12.3%) and low in 135 (30.6%) (See Tables 2 and 3).

In addition, oral leukoplakia and oral submucous fibrosis showed different intracellular expression sites in terms of Snail expression. In the epithelium, Snail expression was shown only in the cytoplasm in the case of healthy individuals as a control, while Snail expression was shown in both in the nucleus and the cytoplasm in the case of 58 of 154 patients with oral leukoplakia (37.7%; P = 0.001) and 13 of 36 patients with oral submucous fibrosis (36.1%; P = 0.001) (See FIGS. 2C and 2F). Overall tissue immunoreactivity for Snail was high in 27 oral leukoplakia samples (17.5%) and low in 127 (82.5%). In contrast, the overall tissue immunoreactivity for Snail was high in 5 oral submucous fibrosis samples and low in 31 (86.1%) (See Tables 2 and 3).

[Table 2]

Changes in clinicopathologic factors and protein expression according to oral leukoplakia malignancy

| Factor | Total number(%) | Oral leukoplakia | | P |
| | | Non-progressed | Progressed | |
| | | n(%) | n(%) | |
|---|---|---|---|---|
| Age(years old) | | | | |
| <55. | 78(50.6) | 72(92.3) | 6(7.7) | 0.018 |
| ≥55 | 76(49.4) | 60(78.9) | 16(21.1) | |
| Gender | | | | |
| Male | 96(62.3) | 86(89.6) | 10(10.4) | n.s. |
| Female | 58(37.7) | 46(79.3) | 12(20.7) | |
| Site | | | | |
| Oral mucosa | 44(28.6) | 38(86.4) | 6(13.6) | |
| Tongue | 42(27.3) | 32(76.2) | 10(23.8) | n.s. |
| Gum | 68(44.2) | 62(91.2) | 6(8.8) | |
| Axin-2 expression | | | | |
| Negative | 47(30.5) | 40(85.1) | 7(14.9) | n.s. |
| Positive | 107(69.5) | 92(86.0) | 15(14.0) | |
| Low | | 118(87.4) | 17(12.6) | n.s. |
| High | 19(12.3) | 14(73.7) | 5(26.3) | |
| Snail expression | | | | |
| Negative | 96(62.3) | 88(91.7) | 8(8.3) | 0.007 |
| Positive | 58(37.7) | 44(75.9) | 14(24.1) | |
| Low | 127(82.5) | 116(91.3) | 11(8.7) | <0.00<0.001 |
| High | 27(17.5) | 16(59.3) | 11(40.7) | |

[Table 3]

Changes in clinicopathologic factors and protein expression according to malignancy of submucous fibrosis

| Factor | Total number(%) | Submucous fibrosis | | P |
| | | Non-progressed | Progressed | |
| | | n(%) | n(%) | |
|---|---|---|---|---|
| Age(years old) | | | | |
| <46 | 18(50.0) | 16(88.9) | 2(11.1) | n.s. |
| ≥46 | 18(50.0) | 15(83.3) | 3(16.7) | |
| Gender | | | | |
| Male | 23(63.9) | 21(91.3) | 2(8.7) | n.s. |
| Female | 13(36.1) | 10(76.9) | 3(23.1) | |
| Site | | | | |
| Oral cavity cheek site | 34(94.4) | 30(88.2) | 4(11.8) | n.s. |
| Upper lip | 2(5.6) | 1(50.0) | 1(50.0) | |
| Axin-2 expression | | | | |
| Negative | 11(30.6) | 10(90.9) | 1(9.1) | n.s. |
| Positive | 25(69.4) | 21(84.0) | 4(16.0) | |
| Snail expression | | | | |
| Negative | 23(63.9) | 21(91.3) | 2(8.7) | n.s. |
| Positive | 13(36.1) | 10(76.9) | 3(23.1) | |

(continued)

| Snail expression | | | | |
|---|---|---|---|---|
| Low | 31(86.1) | 29(93.5) | 2(6.5) | 0.013 |
| High | 5(13.9) | 2(40.0) | 3(60.0) | |

Example 2: Verification of Clinically Pathological Significance of Expression of Axin2 and Snail in Oral Leukoplakia

Mucosal tissue expression of Axin2 and Snail in each of the tissue samples identified in Example 1 was compared using Fisher's exact test and a Chi-square test.

The correlation between Axin2 and Snail was analyzed using a Chi-square test.

All statistical analyses were performed using R package version 3.1.1 of the rms and eha libraries (The R Foundation for Statistical Computing, Vienna, Austria).

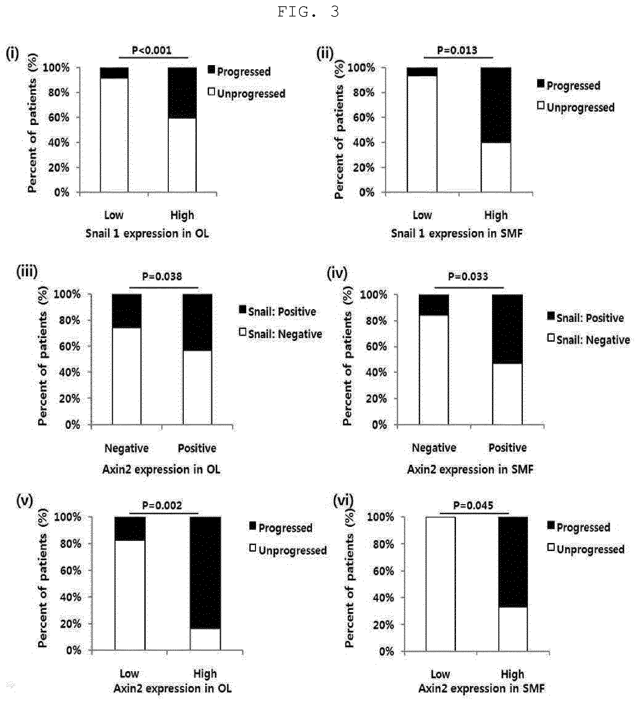

As a result, it was confirmed that none of the basic information of clinical factors including age, gender, and site was related to the expression of Axin2 and Snail in tissues of patients with oral leukoplakia and oral submucous fibrosis. In contrast, malignancy occurred more frequently in oral leukoplakia patients with tissues exhibiting high Snail expression compared to tissues exhibiting low Snail expression (40.7% versus 8.7%; P < 0.001; see FIG. 3A). In addition, among oral submucous fibrosis patients, the possibility of developing malignancy was higher in patients exhibiting high Snail expression in mucosal epithelial cells than in patients exhibiting low Snail expression (60% versus 6.5%; P = 0.013; see FIG. 3B).

No significant association was found between clinical parameters and tissue Axin2 expression in oral submucous fibrosis and oral leukoplakia. Nevertheless, Axin2 expression is significantly associated with Snail1 expression in oral leukoplakia (p = 0.038) and oral submucous fibrosis (P = 0.033) (See FIGS. 3C and 3D). In particular, among Snail1-positive oral leukoplakia patients, a considerably higher percentage of malignancy occurred in high Axin2 oral leukoplakia lesions than in low Axin2 oral leukoplakia lesions (83.3% versus 17.3%; P = 0.002; see FIG. 3E). Similarly, among patients with Snail-positive oral submucous fibrosis, oral squamous cell carcinoma occurred more frequently in a high Axin2 group than in a low Axin2 group (66.7% versus 0%; P = 0.045; see FIG. 3F).

Example 3: Identification of Risk Factors Causing Malignancy in Oral Leukoplakia

Kaplan-Meier survival curves related to Axin2 expression and Snail expression were drawn, and the differences were analyzed by a log-rank test for the assessment of cancer-free survival in patients with oral leukoplakia. In addition, the intensity of association between malignancy of oral leukoplakia and various factors was evaluated using univariate and multivariate analyses using a Cox proportional hazards regression model. Variables with P < 0.15 in the univariate analysis were included in the multivariate analysis, and data were expressed as odds ratios (ORs) and 95% confidence intervals (CI).

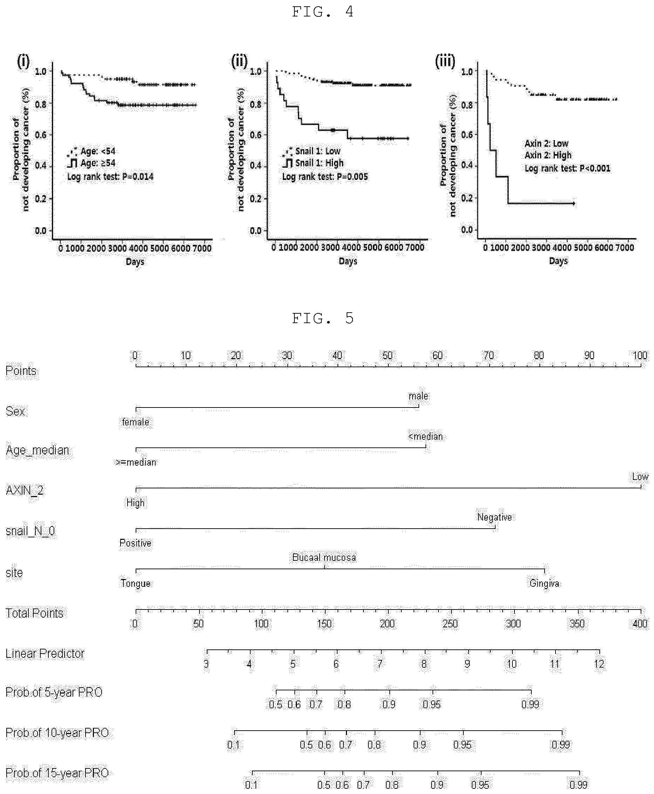

Kaplan-Meier analysis was used to estimate the cancer-free survival rates of oral leukoplakia patients. As a result, low survival rates were exhibited in elderly patients (survival period median-younger age-4427.5 days: older age-3,452 days; P = 0.014) or high Snail expression cases (survival period median-low expression: 4,025 days-high expression: 3,589 days; P = 0.005) (See FIGS. 4A and 4B) .

On the other hand, clinicopathologic variables of P < 0.15 were considered to include multivariate analysis in univariate comparisons of patients with or without malignant mutation (See Table 4). In the univariate analysis, among factors including gender (P = 0.086), age (P = 0.019), Snail expression (P = 0.007), and Axin2 expression (P = 0.066), Cox regression analysis showed that gender (OR = 3.13, 95% CI=1.27-7.73; P = 0.014), age (OR = 3.15, 95% CI = 1.19-8.28; P = 0.02), Snail expression (OR = 4.41, 95% CI = 1.78-10.93; P = 0.001), and Axin2 expression (OR = 7.47, 95% CI = 2.23-25.02; P = 0.001) were significantly associated with the risk of malignancy in oral leukoplakia patients.

In addition, risk factors associated with cancer-free survivability among patients with Snail-positive oral leukoplakia were analyzed. According to Kaplan-Meier analysis, patients with high Axin2 expression during the onset of illness had a low survival rate (survival period median: high Axin2 expression-374 days: low Axin2 expression-3,758 days; P < 0.001 (See FIG. 4C)). Among gender (P = 0.104) and Axin2 expression (P < 0.001) by univariate analysis, it was confirmed through multivariate analysis that a high Axin2 expression level (OR = 23.61, 95% CI = 4.98-111.96; P < 0.001) was considerably associated with malignant modification in oral leukoplakia patients exhibiting positive Snail expression (See Table 5).

[Table 4]

Cox regression analysis for malignancy risk factors of 154 patients with oral leukoplakia

| | Univariate | | Multivariate | |
|---|---|---|---|---|
| Variable | Hazard ratio (95% CI) | P | Hazard ratio (95% CI) | P |
| Gender | | | | |
| Male | 1 | | 1 | |
| Female | 2.09(0.90-4.83) | 0.086 | 3.13(1.27-7.73) | 0.014 |
| Age(years old) | | | | |
| <55 | 1 | | 1 | |
| ≥55 | 3.08(1.20-7.88) | 0.019 | 3.15(1.19-8.28) | 0.02 |
| Site | | | | |
| Oral mucosa | 1 | | 1 | |
| Tongue | 1.76(0.64-4.84) | 0.27 | 2.04(0.71-5.86) | 0.184 |
| Gum | 0.63(0.20-1.95) | 0.42 | 0.39(0.11-1.33) | 0.134 |
| Snail | | | | |
| Negative | 1 | | 1 | |
| Positive | 3.28(1.38-7.83) | 0.007 | 4.41(1.78-1093) | 0.001 |
| Axin2 | | | | |
| Low | 1 | | 1 | |
| High | 2.55(0.94-6.91) | 0.066 | 7.47(2.23-25.02) | 0.001 |

[Table 5]

Cox regression analysis for malignancy risk factors of 58 patients with oral leukoplakia expressing Snail

| | Univariate | | Multivariate | |
|---|---|---|---|---|
| Variable | Hazard ratio (95% CI) | P | Hazard ratio (95% CI) | P |
| Gender | | | | |
| Male | 1 | 0.104 | 1 | 0.054 |
| Female | 2.39(0.84-6.81) | | 3.10(0.98-9.84) | |
| Age(years old) | | | | |
| <55 | 1 | 0.26 | 1 | 0.84 |
| ≥55 | 1.88(0.63-5.61) | | 1.14(0.33-3.92) | |
| Site | | | | |
| Oral mucosa | 1 | | 1 | |
| Tongue | 1.51(0.41-5.62) | 0.54 | 2.15(0.50-9.30) | 0.306 |
| Gum | 1-01(0.27-3.76) | 0.99 | 0.47(0.11-2.04) | 0.315 |
| Axin2 | | | | |
| Low | 1 | <0.001 | 1 | <0.00 |
| High | 11.16(3.58-34.85) | | 23.61(4.98-111.96) | 1 |

Example 4: Nomogram Analysis

Nomograms for predicting risk factors in the development of oral squamous cell carcinoma cells from leukoplakia lesions were created using selected important parameters and evaluated using a concordance index (c-index) and a calibration plot. P-values <0.05 were considered significant.

A nomogram was created to predict 5-year, 10-year, and 15-year cancer-free survival rates using clinical parameters and the expression of Axin2 and Snail1 (See FIG. 5). When the expression of Axin2 and Snail was included, the c-index was 0.760 in the nomogram, and, while the total points decreased, the possibility of progression increased. Because of this, if the patient's total point is 100, the probability of 5, 10, and 15 years without progress is 73%, 60%, and 50%, respectively. On the other hand, if the total point is 236, the likelihood of 5, 10, and 15 years without progress is 95%, 91.5%, and 89%, respectively.

Measurement of the nomogram was performed to investigate the correlation between nomogram prediction and actual results in oral leukoplakia patients. For this, the x-axis represents a predicted value calculated by the nomogram, and the y-axis represents the actual likelihood of disease progression. An ideal nomogram will show a straight line of y = x indicating that the expected nomogram is completely consistent with actual clinical results. Drawing nomogram data results in a solid line reflecting a high level of predictability (See FIG. 6).

In addition, the total points calculated in the nomogram are examined to evaluate characteristics of the predicted malignancy. The total points ranged from 0 to 365, and the patients were divided into three groups using a low quadrant value, i.e., 193, and a high quadrant value, i.e., 294. Thereafter, the Kaplan-Meier survival curves were drawn, and a log-rank test was performed (See FIG. 7). Other important patterns were found in a series of layered curves of the total points measured by the nomogram (P < 0.001). This suggests that Axin2 expression and Snail expression may be used as important markers for predicting malignant progression in oral leukoplakia.

Example 5: Effect of Inhibiting Axin2 and Snail Using Low Molecular Weight Compound

It was examined whether the expression of Axin2 and Snail cancer genes, which promote the cancer progression of precancerous lesions, is effectively inhibited when HCT116, SW480, SW620, and DLD-1 cells (ATCC, American Type Culture collection), which are colorectal cancer cell lines with high Axin2 expression, were treated with niclosamide, which is a low molecular weight compound that inhibits the expression of Axin2 and Snail, at an nM level.

As a result, as illustrated in FIG. 8, it was confirmed that Axin2 expression and Snail expression were decreased upon treatment of niclosamide at an nM level. This suggests that, when niclosamide is formulated in the form of a mouthwash, a solution, a gel, a patch, or the like and topically used, progression of precancerous lesions of a patient to oral cancer may be effectively delayed and inhibited.

Example 6: Inhibition of Axin-GSK3 Complex Formation

To confirm whether niclosamide is capable of inhibiting Axin-GSK3 interaction, cell lysates with full-length Axin2 were examined to perform immunoprecipitation experiments in the presence or absence of niclosamide.

An immunoprecipitation assay was performed as follows (Yook JI, et al. Nat Cell Biol.8:1398-1406, 2006).

A doxycycline-induced His-tagged Axin2 expression vector was transfected into MCF-7 cells (ATCC, American Type Culture collection) and cultured, and a Triton X-100 lysate of whole cells was treated with Ni-Ti beads (Invitrogen) and niclosamide at each concentration to allow a reaction to occur therebetween. Proteins recovered in the beads were subjected to SDS-PAGE, followed by immunoblot analysis for GSK3, and a 1/20 volume of a control was added thereto.

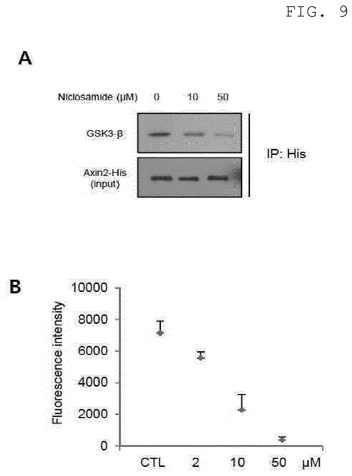

As a result, as illustrated in FIG. 9A, it was confirmed that niclosamide reduced GSK3 that binds to Axin2 in a total cell lysate.

In the previously reported results of analyzing the structure of Axin-GSK3 binding, accumulation of the hydrophobic residues of Axin $\alpha$-helix into a hydrophobic groove formed by the C-terminal loop of GSK3 is disclosed (Dajani R et al., EMBO J. 22:494-501, 2003), and thus the inventors of the present invention hypothesized that niclosamide would bind to the hydrophobic groove of GSK3 and interfere with the function of Axin. To verify this, in vitro analysis was designed to identify competitive inhibition of recombinant GSK3 binding to 19-mer FITC-conjugated Axin peptides by niclosamide.

Hig-tagged recombinant GSK3 beta was obtained from sf9 insect cells using a known method (Lee DG, et al. Nat Commun. 5:4423, 2014). The FITC-conjugated 19-mer Axin peptide (Axin1, 383-401, VEPQKFAEELIHRLEAVQR), which is known to bind to GSK3 as an amphipathic $\alpha$-helix, was chemically synthesized (Peptron). To confirm competitive binding between the synthesized Axin peptide and niclosamide, His-tagged recombinant GSK3 and Ni-Ti beads were treated with niclosamide for 2 hours, and then washed three times with PBS, and quantitative fluorescence measurement was performed using Ni-Ti beads. Fluorescence intensity was represented in proportionate to the fluorescence intensity of a negative control from the 3rd experiment.

As a result, it was confirmed that the interaction between the recombinant GSK3 and the synthesized Axin peptide was inhibited in proportionate to the concentration of niclosamide added (See FIG. 9B). These results indicate that niclosamide binds to GSK-3, thereby inhibiting the function of Axin2 and further inhibiting Snail expression.

Example 7: SPR Analysis and Structural Analysis

Surface plasma resonance (SPR) analysis was performed to confirm whether niclosamide directly binds to GSK3.

SPR was conducted using the ProteOn™ XPR36 Protein Interaction Array system (Bio-Rad Laboratories, Inc., CA, USA), and purified recombinant GSK3$\beta$ was immobilized to a ProteOn GLH sensor chip. Niclosamide or 19-mer wild type Axin peptide or mutant peptide (VEPQKAAEEAIHRAEAVQR, mutation underlined) were diluted by phosphate-buffered saline + Tween 20 + 1% DMSO at different concentrations and then flowed over the chip at a rate of 100 $\mu$l/min. The results thereof were analyzed by ProteOn Manager Software 2.0 using the standard Langmuir models for fitting kinetic data. The rate of complex formation was represented by the association constant (ka, in the unit of M-1s-1), and the rate of complex decay was represented by the dissociation constant (kd, in the unit of s-1), as given by Equation 1 below.

[Equation 1]

$$A+B \overset{ka}{\underset{kd}{\Leftrightarrow}} AB$$

A high-affinity interaction is represented by a low dissociation constant, and rapid recognition and binding with interactants (rapid "on rate," or high $k_a$) and the stability of complex formation (slow "off rate," or low $k_d$) are as shown in the following equation: $K_D = k_d/k_a$.

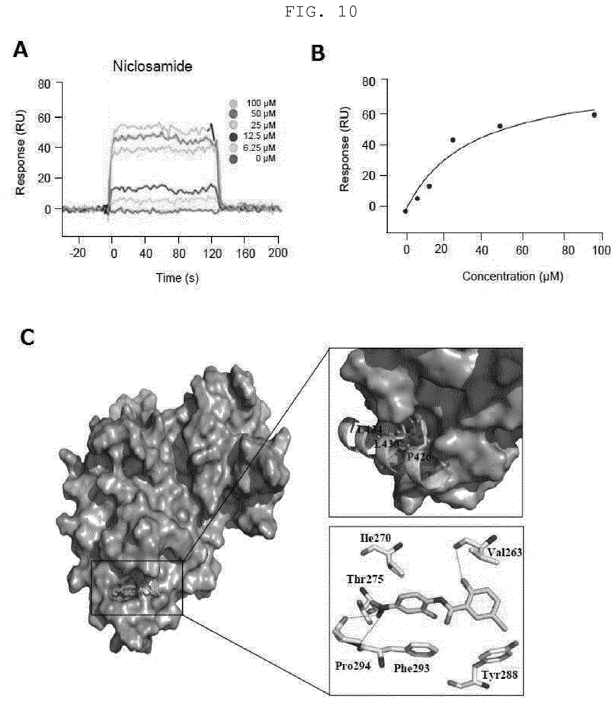

As a result, as illustrated in FIGS. 10A and 10B, wild type Axin peptide or niclosamide was directly bound to GSK3. The equilibrium dissociation constant yields a $K_D$ value of 35 $\mu$M and 34 $\mu$M in SPR analysis, respectively. Mutant Axin peptide having mutations on hydrophobic residues at the GSK3 protein immobilized on a sensor surface was unable to bind up to 100 $\mu$M.

In addition, molecular docking analysis was performed to structurally analyze the interaction between the Axin-binding site of GSK3 and niclosamide.

Molecular docking measurement was performed using the Maestro 10.4 molecular docking suite. The crystal structure of the human (pTyr216)-GSK3$\beta$ bound with an Axin peptide was measured by the RCSB Protein Data Bank (PDB ID: 3ZDI). All water molecules and metal ions were removed, and hydrogen atoms were added to the protein, and the Epik module was used to add protons to a ligand different from a sample at physiological pH. All compounds were energy-minimized using LigPrep and docked to receptor structures using the standard precision (SP) module of the Glide docking module within the Schrödinger Suite. Prior to Glide docking analysis, a receptor grid box was generated at the centroid of co-crystallized ligands. Post-minimization was performed to optimize geometrical forms.

As a result, as illustrated in FIG. 10C, the 1-chloro-3-nitrobenzene group of niclosamide docked into a hydrophobic cavity formed by residues Val 263, Leu 266, Val 267, and Ile 270 of human GSK3$\beta$ and was stacked onto residue Phe 293 through $\pi$-$\pi$ interactions. Niclosamide additionally forms hydrogen bonds with Pro294, Thr275, and Val 263, and halogen bonds with Tyr288 on the Axin-GSK3 surface. From these results, it was confirmed that niclosamide disrupted the Axin-GSK3 complex by inhibiting protein-protein interactions (PPI).

Example 8: Reducing Snail Expression Using 2'-Hydroxycinnamaldehyde (HCA) and 2'-benzoyloxycinnamaldehyde (BCA)

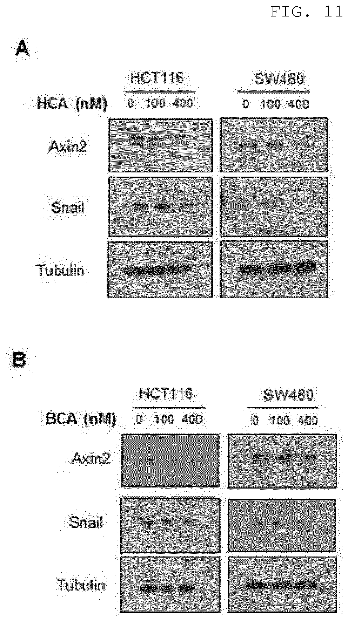

According to the previous research results, it has been known that 2'-Hydroxycinnamaldehyde (HCA), which is a main component of cinnamon, and a derivative thereof, i.e., 2'-benzoyloxycinnamaldehyde (BCA) inhibit cancer metastasis through Snail expression inhibition by inhibiting LRP-1 receptors (Hwang H et al., J Neuroimmunol 2011, 230, 52-64; Ismail IA, et al., Breast Cancer Res Treat, 137, 697-708, 2013). Thus, to confirm whether HCA and BCA are capable of inhibiting Axin2 expression and Snail expression, colorectal cancer cells were treated with HCA and BCA, and it was examined whether Axin2 and Snail were expressed in a cell lysate. As HCA and BCA, those separated from cinnamon bark and purified were used (Hwang H et al., J Neuroimmunol 2011, 230, 52-64). As a result, as illustrated in FIGS. 11A and 11B, it was confirmed that HCA and BCA inhibited the expression of Axin2 and Snail at an nM level. These results suggest that HCA and BCA may be used for inhibiting or treating the progression of oral precancerous lesions.

[Industrial Applicability]

According to the present invention, the possibility of developing malignancy such as into oral cancer from oral leukoplakia or oral submucous fibrosis with a white lesion in the mouth may be predicted and diagnosed, and thus the possibility of developing into oral precancer and oral cancer, which has no effective treatment method so far, is predicted early, thereby effectively removing the potential risk of oral cancer. The present invention also provides an oral therapeutic agent capable of controlling the progression and recurrence of precancerous lesions and the recurrence of oral cancer by providing drug information and effects for effectively controlling cancer genes that contribute to oral cancer malignancy, thus providing clinical benefits to patients.

While the present invention has been particularly shown and described with reference to specific embodiments thereof, it will be obvious to those of ordinary skill in the art that such specific embodiments are provided for illustrative purposes only and are not intended to limit the scope of the present invention. Therefore, the true scope of the present invention should be defined by the appended claims and equivalents thereto.

**Claims**

1.  A method of providing information for the prediction or diagnosis of oral precancer or oral cancer, the method comprising confirming an expression level of an Axin2 gene or a Snail gene.

2.  The method according to claim 1, wherein the confirming of the expression level of an Axin2 gene or a Snail gene is performed in an oral leukoplakia tissue sample or an oral submucous fibrosis tissue sample.

3.  The method according to claim 1, wherein the confirming of the expression level of an Axin2 gene or a Snail gene is performed using a method selected from the group consisting of RT-PCR, immunohistochemical staining, and enzyme-linked immunosorbent assay (ELISA).

4.  A composition for predicting or diagnosing oral precancer or oral cancer, the composition comprising an agent for confirming expression of an Axin2 gene or a Snail gene.

5.  The composition according to claim 4, wherein the agent for confirming expression of an Axin2 gene or a Snail gene is selected from the group consisting of an antibody or aptamer that specifically binds to an Axin2 protein or a Snail protein, a primer that specifically amplifies the Axin2 gene or the Snail gene, and a probe specifically binding to the Axin2 gene or the Snail gene.

6.  A kit for predicting or diagnosing oral precancer or oral cancer, the kit comprising an agent for confirming expression of an Axin2 gene or a Snail gene.

7.  The kit according to claim 6, wherein the agent for confirming expression of an Axin2 gene or a Snail gene is selected from the group consisting of an antibody or aptamer that specifically binds to an Axin2 protein or a Snail protein, a primer that specifically amplifies the Axin2 gene or the Snail gene, and a probe specifically binding to the Axin2 gene or the Snail gene.

8.  A pharmaceutical composition for delaying or inhibiting progression of an oral precancerous lesion and recurrent oral cancer, the pharmaceutical composition comprising, as an active ingredient, a compound for inhibiting expression of an Axin2 gene or a Snail gene.

9.  The pharmaceutical composition according to claim 8, wherein the compound for inhibiting expression of an Axin2 gene or a Snail gene is selected from the group consisting of niclosamide, 2'-hydroxycinnamaldehyde (HCA), 2'-benzoyloxycinnamaldehyde (BCA) as a derivative of HCA, and a pharmaceutically acceptable salt thereof.

FIG. 1

FIG. 2

FIG. 3

FIG. 4

(i) Proportion of not developing cancer (%) — Age: <54 / Age: ≥54 / Log rank test: P=0.014

(ii) Proportion of not developing cancer (%) — Snail 1: Low / Snail 1: High / Log rank test: P=0.005

(iii) Proportion of not developing cancer (%) — Axin 2: Low / Axin 2: High / Log rank test: P<0.001

FIG. 5

FIG. 6

FIG. 7

FIG. 8

FIG. 9

**A**

Niclosamide (µM)   0    10    50

GSK3-β

Axin2-His
(input)

IP: His

**B**

FIG. 10

FIG. 11

A

HCT116    SW480

HCA (nM)   0  100  400    0  100  400

Axin2

Snail

Tubulin

B

HCT116    SW480

BCA (nM)   0  100  400    0  100  400

Axin2

Snail

Tubulin

## INTERNATIONAL SEARCH REPORT

| | International application No. |
|---|---|
| | **PCT/KR2018/000395** |

**A. CLASSIFICATION OF SUBJECT MATTER**

*C12Q 1/68(2006.01)i, G01N 33/574(2006.01)i, G01N 33/68(2006.01)i, A61K 31/11(2006.01)i, A61K 31/167(2006.01)i, A61K 48/00(2006.01)i*

According to International Patent Classification (IPC) or to both national classification and IPC

**B. FIELDS SEARCHED**

Minimum documentation searched (classification system followed by classification symbols)

C12Q 1/68; G01N 33/574; G01N 33/68; A61K 31/11; A61K 31/167; A61K 48/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: oral cancer, oral precancer, Axin2, Snail, expression

**C. DOCUMENTS CONSIDERED TO BE RELEVANT**

| Category* | Citation of document, with indication, where appropriate, of the relevant passages | Relevant to claim No. |
|---|---|---|
| X | RYU, Mi Heon et al., "The Immunohistochemical Expression of Snail and E-cadherin in Oral Squamous Cell Carcinoma", Kor. J. Oral Maxillofac. Pathol., 2009, vol. 33, no. 1, pages 37-46 See abstract; page 39, right column-page 40. | 1-8 |
| Y | | 1-8 |
| Y | YOOK, J. I. et al., "A Wnt-Axin2-GSK3beta Cascade Regulates Snail1 Activity in Breast Cancer Cells", Nature Cell Biology, December 2006, vol. 8, no. 12, pages 1398-1406 See abstract; figures 1-2. | 1-8 |
| X | AHN, S.-G. et al., "The Anticancer Mechanism of 2'-hydroxycinnamaldehyde in Human Head and Neck Cancer Cells", International Journal of Oncology, 2015, vol. 47, pages 1793-1800 See abstract; page 1794, left column, page 1795; figures 2-4. | 9 |
| X | KIM, S.-A et al., "2'-Hydroxycinnamaldehyde Shows Antitumor Activity against Oral Cancer In Vitro and In Vivo in a Rat Tumor Model", Anticancer Research, 2010, vol. 30, pages 489-494 See abstract; pages 491-492; figures 1-3. | 9 |
| A | KR 10-2015-0125030 A (INDUSTRY-ACADEMIC COOPERATION FOUNDATION, YONSEI UNIVERSITY) 09 November 2015 See the entire document. | 1-9 |

☐ Further documents are listed in the continuation of Box C.   ☒ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 27 APRIL 2018 (27.04.2018) | **27 APRIL 2018 (27.04.2018)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

EP 3 567 119 A1

INTERNATIONAL SEARCH REPORT
Information on patent family members

International application No.

**PCT/KR2018/000395**

| Patent document cited in search report | Publication date | Patent family member | Publication date |
|---|---|---|---|
| KR 10-2015-0125030 A | 09/11/2015 | NONE | |

Form PCT/ISA/210 (patent family annex) (January 2015)

27

## REFERENCES CITED IN THE DESCRIPTION

### Patent documents cited in the description

- US 4683195 A
- US 4683202 A
- US 4800159 A
- WO 8906700 A, Miller, H. I.
- EP 329822 A, Davey, C.
- WO 9001069 A
- EP 439182 A
- WO 8810315 A
- WO 9006995 A
- US 6410276 B
- US 4437975 A
- US 5413909 A
- US 5861245 A
- US 5130238 A
- US 5409818 A
- US 5554517 A
- US 6063603 A
- US 5242794 A
- US 5494810 A
- US 4988617 A
- US 09854317 B

### Non-patent literature cited in the description

- **VAN DER WAAL I.** *Med Oral Patol Oral Cir Bucal.,* 2014, vol. 19, e386
- **HOLMSTRUP P et al.** *Oral Oncol,* 2006, vol. 42, 461
- **HOLMSTRUP P.** *Oral Oncol,* 2009, vol. 45, 549
- **ABDULRAHIM MH et al.** *PLoS One,* 2013, vol. 8, e73738
- **ZHANG L et al.** *Cancer Res,* 2005, vol. 65, 8017
- **NASSER W et al.** *J Oral Pathol Med,* 2011, vol. 40, 629
- **SIEBERS TJ et al.** *Oral Oncology,* 2013, vol. 49, 1121
- **GRAVELAND AP et al.** *Oral Oncol,* 2013, vol. 49, 1129
- **DANG TT et al.** *Cancer Res,* 2015, vol. 75, 3925
- **VON ZEIDLER SV et al.** *BMC Cancer,* 2014, vol. 14, 972
- **SLAUGHTER DP et al.** *Cancer,* 1953, vol. 6, 963-968
- **MOHAN M ; JAGANNATHAN N.** *Oncology Rev,* 2014, vol. 8 (13-19), 244
- **CARVER EA et al.** *Mol Cell Biol,* 2001, vol. 21, 8184
- **ZHOU BP et al.** *Nat Cell Biol,* 2004, vol. 6, 931-40
- **BACHELDER RE et al.** *J Cell Biol,* 2005, vol. 168, 29
- **YOOK JI et al.** *J Biol Chem,* 2005, vol. 280, 11740
- **KLAR M et al.** *Breast Cancer Res Treat,* 2008, vol. 112, 523
- **SAMBROOK, J. et al.** Molecular Cloning. A Laboratory Manual. Cold Spring Harbor Press, 2001
- **TESNIERE, C. et al.** *Plant Mol. Biol. Rep.,* 1991, vol. 9, 242
- **AUSUBEL, F.M. et al.** Current Protocols in Molecular Biology. John Willey & Sons, 1987
- **CHOMCZYNSKI, P. et al.** *Anal. Biochem.,* 1987, vol. 162, 156
- *PNAS USA,* 1988, vol. 85, 8998
- **LIBERT F et al.** *Science,* 1989, vol. 244, 569
- **JOSEPH SAMBROOK et al.** Molecular Cloning, A Laboratory Manual. Cold Spring Harbor Laboratory Press, 2001
- A Practical Approach. **HAYMES, B.D. et al.** Nucleic Acid Hybridization. IRL Press, 1985
- **SAMBROOK et al.** Molecular Cloning. A Laboratory Manual. Cold Spring Harbor Press, 2001
- **MCPHERSON, M.J. ; MOLLER, S.G.** PCR. BIOS Scientific Publishers, 2000
- **MAXAM ; GILBERT.** *Methods in Enzymology,* 1986, vol. 65, 499
- **M.L.M. ANDERSON.** Nucleic Acid Hybridization. Springer-Verlag, 1999
- **YOOK JI et al.** *Nat Cell Biol.,* 2006, vol. 8, 1398-1406
- **DAJANI R et al.** *EMBO J.,* 2003, vol. 22, 494-501
- **LEE DG et al.** *Nat Commun.,* 2014, vol. 5, 4423
- **HWANG H et al.** *J Neuroimmunol,* 2011, vol. 230, 52-64
- **ISMAIL IA et al.** *Breast Cancer Res Treat,* 2013, vol. 137, 697-708